# EUROPEAN PATENT APPLICATION

(11) **EP 3 543 723 A1**
(43) Date of publication of application: **25.09.2019**
(21) Application number: 18162807.4
(22) Date of filing: 20.03.2018
(51) Int. Cl.: G01R 33/48, G01R 33/54, A61N 5/10

(54) **METHOD FOR ACQUIRING MAGNETIC RESONANCE IMAGE DATA FOR IMAGE-GUIDED RADIOTHERAPY**

(71) Applicant: Siemens Healthcare GmbH, 91052 Erlangen (DE)
(72) Inventor: Nioutsikou, Elena, 91056 Erlangen (DE)

(57) **Abstract**

The invention relates to a method for acquiring magnetic resonance image data (D) for image-guided radiotherapy, wherein a number of imaging goals (U) are provided, wherein for each imaging goal (U) a magnetic resonance imaging protocol (R)is selected, wherein for each selected magnetic resonance imaging protocol (R) a number of parameters (A) for said protocol (R) is adjusted according to the respective imaging goal (U), and wherein the magnetic resonance image data (D) are acquired with said magnetic resonance imaging protocols (R) and with said parameters (A).

## Description

The present invention relates to a method for acquiring magnetic resonance image data for image-guided radiotherapy Further the present invention relates to a processing device for executing said method, a database system with an interface for such a processing device and a computer program product.

Radiation therapy (radiotherapy, RT) is typically used to treat a patient's disease, in particular cancer. For that purpose, a target volume comprising tumor cells or malignant cells is irradiated with ionizing radiation. Within the treatment, the radiation therapy is either used by itself or in combination with other forms of treatment such as chemotherapy or surgery. Furthermore, the ionizing radiation is either delivered from outside the patient's body (external-beam radiation therapy) or, alternatively, a radiation source is placed within the patient's body (internal radiation therapy, brachytherapy), in particular in the target volume.

In order to avoid the irradiation of tissue around the target volume, in particular organs at risk, and in order to deliver a radiation dose into the target volume, which dose is sufficient to kill or damage the tumor cells or the malignant cells, preferably 3-dimensional medical images of the patient and especially of the target volume are used to plan the irradiation. For this so called image-guided radiotherapy, those medical images are acquired by means of a x-ray computer tomography-scanner (CT-scanner), exemplarily.

Preferably however, those medical images are acquired by means of a magnetic resonance imaging scanner (MRI-scanner). The use of an MRI-scanner provides a comparatively high soft tissue contrast, which for example facilitates a classification of cancer tissue and the determination of the shape for delineation of the target and/or of an organ at risk (OAR).

Acquiring a magnetic resonance image and respectively image data, by which means the image is reconstructed, for a given imaging goal (purpose), such as the determination of an organ at risk delineation, the study of a tumor diffusion or a motion analysis is a comparatively complex task. This is due to the fact that a number of (magnetic resonance imaging) protocols can be used for acquiring magnetic resonance image and respectively image data for a given imaging goal. Furthermore the appearance and characteristics, e.g. a slice thickness, a resolution, or a contrast to noise ratio, of said magnetic resonance image varies for each protocol and for parameters such as a field of view (FOV), a receiver bandwidth or a matrix size, used within said protocol.

On the one hand, the education of radiation therapy staff, such as radiation oncologists or physicists, typically doesn't involve sufficient training in magnetic resonance imaging. On the other hand radiologist, who are trained in magnetic resonance imaging, typically have no or not sufficient education in radiation therapy. Consequently, protocols and/or the parameters can be used for acquiring a magnetic resonance image or image data, which are not suited for the respective imaging goal, and an (systematic) error can be introduced in the image-guided radio therapy process. This can result in a less effective treatment of the patient's disease and, consequently, in a less effective or even detrimental therapeutic result.

MR-based methods to support the planning of the irradiation of a patient are e.g. known from US 2016/0082285 A1 or US 9878178 B2.

It is an object of the present invention to support the planning of the irradiation of a patient in radiation therapy by means of magnetic resonance imaging. Furthermore, it is an object of the present invention to provide a processing device for executing said method, a database system providing information for said method and a respective computer program product.

According to the invention, in view of supporting the planning the irradiation said object is achieved by a method with the characteristics of claim 1. In view of the processing device, the object is achieved according to the invention by the characteristics of claim 8, in view of the database system the object is achieved by the characteristics of claim 9 and in view of the computer program product the object is achieved by the characteristics of claim 10. Advantageous developments and embodiments are described in the dependent claims and in the following specifications.

The method is used for acquiring magnetic resonance image data for image-guided radiotherapy. Exemplarily, by means of said magnetic resonance image data, hereinafter simply referred to as image data, an (magnetic resonance) image or a number of images can be reconstructed.

Within a first step of the method, a number of imaging goals (purposes, objects) are provided. In particular, said imaging goals are provided by a user such as radiation therapy staff. Herein, a number of imaging goals is understood to be one imaging goal or more than one imaging goal.

In a second step, for each imaging goal a magnetic resonance imaging protocol, hereinafter simply referred to as protocol, is selected. Therein, in particular, said protocol complies with the respective imaging goal. With other words, the selected protocol is suited to accomplish (fulfill) the respective imaging goal. In a third step for each selected protocol, a number of parameters for said selected protocol are adjusted according to the respective imaging goal. Subsequently, in a fourth step, the magnetic resonance image data are acquired with said protocols and with said parameters.

Preferably, within the fourth step, post-acquisition activities such as distortion correction, reconstruction of images to a transverse plane, are performed. For example, said images are then used in a treatment planning system, in particular for image-guided radiotherapy, or in a contouring system to determine a contour of a target such as a tumor.

A protocol comprises a number of so called sequences, wherein a number of sequences is understood to mean one sequence or more than one sequence. The sequences are a particular setting of chronological order of pulses of radio waves and pulsed field gradients for the acquisition of image data, by means of which the respective image is determined or reconstructed. In particular, the respective image especially is a three-dimensional volume representation of the examined volume or a two-dimensional plane (slice) representation of the examined volume. An appearance of the determined or reconstructed image varies dependent of the used sequence. Exemplarily, the appearance of fatty tissue in the respective reconstructed image is comparatively light for a T1-weighted sequence and comparatively dark for a T2-weighted sequence, respectively.

In particular, protocols can be combined if they share sequences and parameters. Advantageously, by this means, a total time necessary for the acquisition of the image data for all respective imaging goals is reduced.

The parameters are selectable or adjustable quantities either of the sequence or of the image reconstructed by the image data. Exemplarily, the parameter is a receiver bandwidth, a flip angle in a sequence, an acquisition time for a sequence, a field of view, a matrix size of the acquired image or a number of slices.

The imaging goal does not comprise information about a protocol or parameters used for acquiring image data for image-guided radiotherapy. In particular, the imaging goal is a purpose, object or objective, which the image and the respective image data should fulfill (satisfy), such that the planning of the image-guided radiation therapy is supported or facilitated. The imaging goal is provided by the radiation oncology staff, especially. Exemplarily, the imaging goal is the determination of a target delineation, the determination of organ at risk delineation, the determination of brachytherapy applicator delineation or an analysis of target motion. The imaging goal is selected from a list, or provided as a plain text file, exemplarily, and is further provided to a processing device.

Advantageously, by this method it is comparatively simple, especially for an inexperienced user of a magnetic resonance imaging scanner (MRI-scanner), to select and use a protocol and parameters for said protocol, which are suited to comply with the imaging goal. Consequently, systematic errors in the planning and execution of image-guided radiotherapy are avoided, the errors arising in particular from a lack of training in the use of MRI-scanners for radiation therapy.

In an expedient embodiment, a database is provided. The database comprises information, assigning a magnetic resonance imaging protocol and a number of the parameters for said protocol to each of a number of imaging goals. In other words, to each of a number of imaging goals a protocol and parameters for said protocol are linked. In particular, the database comprises a list of imaging goals, wherein a protocol, preferably one, or an identification for a protocol is linked to each imaging goal in the list. Further, the parameters for said protocols are linked to the respective protocols in the database. Especially preferred, said information has been obtained empirically. Exemplarily, said information has been obtained by experienced staff, who used the protocol and the parameters for the protocol for fulfilling the respective imaging goal. Consequently, the expertise and knowledge of said members of staff concerning the use of MRI-scanners is available to other user by means of the database.

In an advantageous embodiment, within the second step, the protocol is selected dependent on the respective imaging goal by means of the database. For this purpose the database is searched for the respective imaging goal and the protocol assigned (linked) to said imaging goal is returned. In particular, by this means, the protocol complies with (fulfills) the imaging goal. Thus, the protocol complying with the respective imaging goal is selected merely by means of the imaging goal.

Preferably, within selecting the protocol, after the protocol is returned from the database, the user confirms or discards the selection of the respective protocol. Additionally, when more than one protocol is returned for a given imaging goal, the user is offered a choice between those protocols.

Within the third step, i.e. within adjusting the parameters for the respective protocol for the given imaging goal, each imaging goal is converted (translated) into a number of first metrics and a range (interval) for each of the first metrics. Therein, a first metric represents a measure (quantity),, which especially is determinable by means of the image, wherein the first metric quantitatively assesses (evaluates, describes) the respective imaging goal. Exemplarily, the first metric is a contrast to noise ratio (CNR) between target and surrounding tissue, a slice thickness of the image or an in-plane-resolution. The range for the first metric is for example expressed by means of a maximum or a minimum value of said first metric. Exemplarily, a range for the slice thickness ranging from of a minimally feasible value, such as 0.1 mm, to 2 mm is expressed by a maximum value of 2 mm for the slice thickness. Exemplarily, for the conversion, a table is provided, which assigns an imaging goal to one first metric and a range for said first metric. Said table is in particular stored in the database. In particular, a user can provide limits for the range of the first metrics, additionally. Exemplarily the user sets manually a maximum slice thickness of 1 mm, in particular by means of an user interface, which is interfaced to the processing device.

According to a further development, boundary conditions are provided and the range of each of the first metrics, which were obtained by converting the respective imaging goals, is adjusted to said boundary conditions. The boundary conditions represent in particular available equipment or patient characteristics. By means of said boundary conditions limits (restrictions) for the range of the first metrics are derived. Adjusting the range of the first metric in other words means, that limits corresponding to the boundary conditions are applied to the range of the respective first metric. Exemplarily, the boundary conditions are selected from a list, or provided as a plain text file, and are further provided to the processing device. Exemplarily, the boundary conditions representing available equipment, are for example a type or a number of coils, a number of available channels, what kind of MRI-scanner is used for acquiring the image data, the magnet strength of the MRI-scanner, or alternatively or additionally the boundary conditions represent patient tolerance on (scan) acquisition time or missing anatomy of the patient.

Furthermore, within the third step of the method for acquiring image data for image-guided radiotherapy, the parameters, which are assigned to the respective protocol in the database, are obtained from the database. In other words the parameters are retrieved from the database, wherein the said parameters are linked to the respective protocol and imaging goal.

Subsequently, a second metric is determined as a function of said parameters. Alternatively, a second metric and a range for the second metric is determined. Therein the second metric represents an expected first metric for using said parameters for acquiring the respective image data. With other words, the second metrics are first metrics, which are predicted or forecast for obtaining (acquiring) image data with said parameters. In particular for determining a second metric, it is calculated by means of physical models, physical laws and/or are predicted by simulations. Preferably, for each first metric one respective second metric is determined. Thus, the number of first metrics and the number of second metrics are equal. Exemplarily, for a contrast to noise ratio as a first metric, a contrast to noise ratio is determined as a second metric.

Subsequently, the range of each of the first metrics is compared to the respective second metric or, alternatively, to the range of the respective second metric. By comparing the range of the first metric to the respective (predicted) second metric, it is possible to verify if parameters for a protocol comply with the imaging goal and the boundary conditions. If the range of one of the first metrics and the respective second metric do not comply, i.e. if the value determined for second metric is not within the range of the respective first metric, or if an range determined for the second metric does not overlap with the range of the respective first metric, said parameters are changed. Alternatively, the paramaters are only changed, if a number of second metrics do not comply with the range of the respective first metric. Preferably, the parameters are changed according to an iterative, non linear optimization-algorithm. Exemplarily, an algorithm using Newton's method or an algorithm using a Quasi-Newton method is used.

Alternatively, parameters are changed according to a machine learning algorithm or according to an artificial neural network or are determined by means of such a machine learning algorithm or by means of such an artificial neural network.

In Particular, this third step is necessary due to the fact, that the parameters retrieved from the database have been used to satisfy certain boundary conditions, which can differ from the current boundary conditions.

In summary, the parameters for the selected protocols are adjusted such that they comply with the imaging goal, wherein boundary conditions are taken into account. Preferably, the user confirms or alters the determined parameters. Additionally, the selected protocol and the parameters for said protocol can be stored, e.g. in the database and by that means used for follow-up examination of the patient without having to select the protocol or adjusting the parameters. Thus, a reproducibility and a comparability of the examinations of the patient is facilitated.

According to an advantageous development of the method for acquiring image data for image- guided radiotherapy, information, which assigns (selected) protocols and (adjusted) parameters used for acquiring the magnetic resonance image data to the respective imaging goal, is stored in the database.

By this means the database is extended. In particular, by means of the information stored in the database, a second alternative protocol and parameters for said second protocol are assigned to the respective imaging goal. Consequently, there are more than one protocol assigned to the respective imaging goal. Exemplarily, the database comprises further assigning the respective protocol to an institution or to a person, who stored the information. It is then possible for a user of the database to choose between alternative protocols for one imaging goal based on the institution or person, who has stored the respective information. Exemplarily, a user is offered to store information in the database based on the quality of the resulting image.

In an expedient embodiment, a processing device comprises a controller for executing the method described above. The processing device in particular is a single work station or a personal computer. Alternatively, the processing device is a server or comprises one or more clustered computing devices. The processing device further comprises an interface to the MRI-scanner. Thus, it is possible to use selected protocol and the parameters for said protocols for the acquisition of the image data by means of said MRI-scanner.

In an expedient development a database system comprises a computer readable storage device, on which the database is stored. Therein the database comprises information, which assign a magnetic resonance imaging protocol and parameters for said protocol to each of a number of imaging goals.

Furthermore, the database system comprises an interface to the processing device. Therein, the database system preferably is or comprises a server. The database system is for example used within one institution or one department of said institution. Consequently, the database, and in particular the extension of the database by storing information, is only available within said institution or department. Alternatively, the database system is used for a number of institutions. With other words, the database system is a shared (global) database system. Therein for example, the name or an identification of the institution, which created and/or used the protocol and the parameters for the protocols for a given purpose and additionally or alternatively stored the respective information in the database , is linked to said information in the database. Consequently, it is possible for a user of the database system to choose a protocol and the parameters for the protocol for a given imaging goal dependent on the name of the institution, which created and/or stored the information in the database.

In an expedient embodiment, a computer program product, which is loadable into a computer readable storage device of the processing device, comprises program code means for executing the method described above, in particular, when executed by the controller of said processing device. Therein, the computer program product is a computer program or comprises a computer program. Expediently, the computer program product is stored on a computer readable storage device, such as a hard disk, an USB-stick. Alternatively, the computer program product is stored on a server and loadable to the computer readable storage device of the processing device from the server.

In the following, an embodiment of the present invention is explained with the reference to the attached drawing.
- FIG 1: shows a flowchart diagram schematically illustrating an embodiment of the method for acquiring magnetic resonance image data for image-guided radiotherapy, wherein a number of imaging goals are provided and for each imaging goal a magnetic resonance imaging protocol is selected and parameters for said protocols are determined, and
- FIG 2: shows magnetic resonance imaging scanner for acquiring magnetic resonance image data, wherein the magnetic resonance imaging scanner is connected to a processing device comprising a controller as well as an interface to a database system.

Corresponding parts are provided with the same reference numerals in all figures.

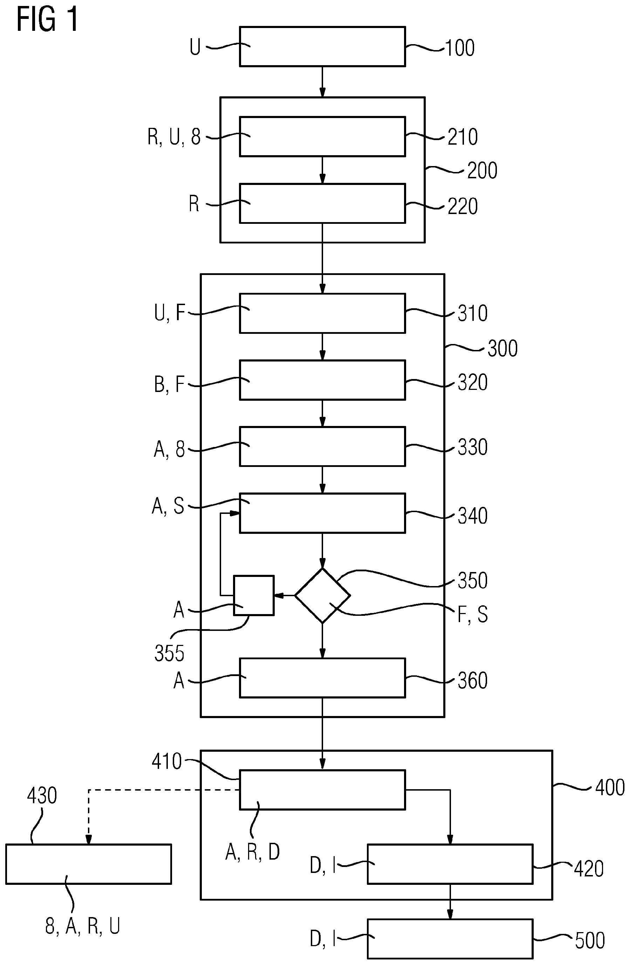

The flowchart diagram of Fig 1 illustrates a method for acquiring (magnetic resonance) image data D for image guided radiotherapy, wherein the image data are used for the reconstruction of one (magnetic resonance) image I or a number of images I.

Within a first step 100 of the method, a number of imaging goals U are provided. Said imaging goals U are provided by a user such as a radiation oncologist. Herein, a number of imaging goals U is understood to be one imaging goal U or more than one imaging goal U. The imaging goal U is selected from a list. For this purpose a processing device 2 comprises a displaying device 4 and an input device 6, which according to this embodiment are a computer screen and a keyboard. Exemplarily, the imaging purpose U is the determination of a tumor delineation of the gross target volume (GTV), an organ at risk delineation of bladder, rectum and pelvic bones or a synthetic CT generation.

In a second step 200, for each imaging goal U a magnetic resonance imaging protocol (protocol R), is selected, wherein the protocol R is suited to accomplish the imaging goal U. For this purpose, a database 8 is provided, wherein the database 8 comprises information, by means of which to each of a number of imaging goals U a protocol R and parameters A for said protocol R are assigned. According to this embodiment, the database 8 comprises a list of imaging goals U, wherein a protocol R, preferably one, is linked to each imaging goal U in the list. Further, the parameters A for said protocols R are linked to the respective protocols R in the database 8.

Within the second step 200, the database 8 is searched for the respective imaging goal U and the protocol assigned (linked) to said imaging goal U is returned (step 210). In summary, the protocol R complying with the respective imaging goal U is selected merely by means of the imaging goal U. Exemplarily, for the imaging goal U "the determination of a target delineation of the gross target volume (GTV)" a protocol comprising the sequence "T2 SPACE 3D" is selected.

Subsequently within the second step 200, the user confirms or discards the selection of the respective protocol R (step 220). In case that more than one protocol R is returned for a given imaging goal U, the user chooses between said protocols R.

Each protocol R comprises a number of sequences, wherein a number of sequences is understood to mean one sequence or more than one sequence. In a not shown alternative embodiment of the method, protocols R are combined subsequently to step 220, if they share sequences and parameters.

In a third step 300, for each selected protocol R, a number of parameters A for said selected protocol R are adjusted according to the respective imaging goal U.

Within this third step 300, each imaging goal U is converted into a number of first metrics F such as a value of a (isotropic) voxel size or a value of a slice thickness and into a range for each of the first metrics F (step 310).

In a not shown alternative, the user manually provides limits for the range of the first metrics F by means of the input device 6 and the displaying device 4.

Subsequently, in step 320, boundary conditions B are provided and the range of each fist metric is adjusted dependent on said boundary conditions B. For this purpose, limits (restrictions) for the range of the first metrics F are derived from the boundary conditions B. Said limits are applied to the respective first metric F. The boundary conditions B are selected from a list by means of the input device 6 and the displaying device 4 of the processing device 2 and provided to the processing device 2.

In a not shown alternative embodiment, steps 310 and 320 are executed between the first step 100 and the second step 200.

Furthermore, within the third step 300, the parameters are retrieved from the database, wherein those parameters A are retrieved from the database 8 (step 330), which are linked to the respective selected protocol R and imaging goal U. In a not shown alternative embodiment of the invention, said parameters A are retrieved from the database 8 within step 200.

In a subsequent step 340, for each first metric F a corresponding second metric S is determined as a function of the retrieved parameters A, the second metrics S are the result of a calculation or prediction of the first metrics F by means of physical models, physical laws or a simulation for obtaining image data D with said parameters A and the corresponding protocol R.

In a comparing step 350, the range of each of the first metrics F is compared to the respective second metric S. If the range of the first metric F and the calculated respective second metric S do not comply, the parameters A are changed (step 355) and the second metrics S are determined as a function of the changed (altered) parameters A. For this purpose, an optimization algorithm using Newton's method or alternatively another optimization algorithm, which for example uses a Quasi-Newton method, is used.

According to not shown alternative embodiments, the parameters are changed according to a machine learning algorithm or by means of a neural network.

In a subsequent step 360, the parameters A are confirmed by the user or alternatively are changed manually by said user. However, by means of the displaying device 4, the user is given instructions, which parameters A should be kept at a fixed value or within what range the parameter A can be changed such that the imaging goal U is still accomplished.

In a fourth step 400, the magnetic resonance image data D are acquired with said protocols R and with said parameters A (step 410). Subsequently, post-acquisition activities such as distortion correction or the reconstruction of images I, are performed by the processing device 2 (step 420).

In an facultative step 430, information, which assigns protocols R and parameters A used for acquiring the magnetic resonance image data D to the respective imaging goal U, is stored in the database 8. By this means the database 8 is extended.

In a fifth step 500, the image data D and/or the reconstructed images I are used for planning an irradiation of a patient. For this purpose said image data D and/or the images I are provided to a radiation oncologist or alternatively or additionally to a planning system.

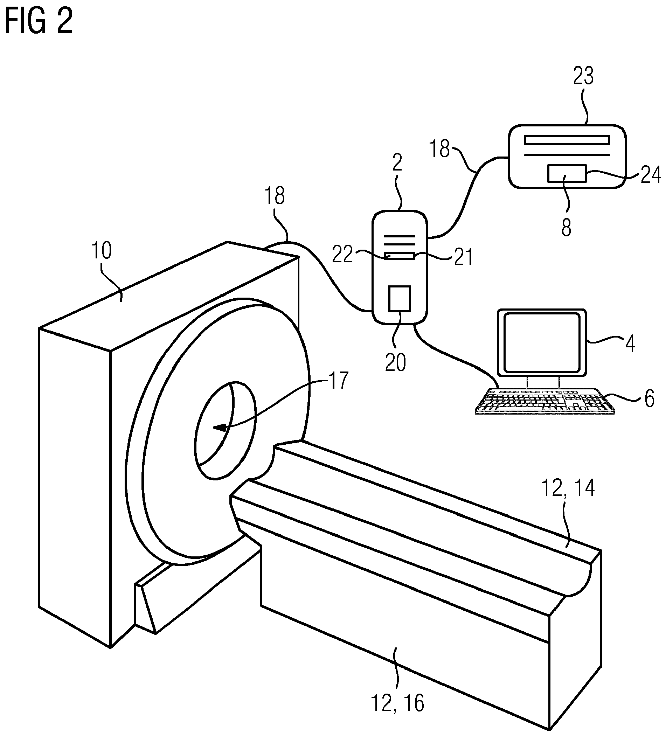

Fig 2 shows a magnetic resonance imaging scanner (MRI scanner) 10, by which means the image data D and images I of a patient are acquired. The images data D are acquired by a method illustrated by the flowchart diagram of FIG 1. Further, a patient table 12 is shown, wherein a top plate 14 of the patient table 12 is traversable relative to a base 16 of the patient table. By this means, a patient lying on the patient table 12 can be brought into a tunnel-shaped acquisition area 17 of the MRI-scanner 10.

The MRI-scanner 10 is further connected to the processing device 2. With other words, the processing device 2 comprises an interface 18 to the MRI-scanner 10. By this means it is possible, to carry out the acquisition of the image data D according to the method described in FIG 1, i.e. the MRI-scanner 10 performs the acquisition of the image data D with the protocols R selected by said method and with adjusted parameters A for the protocols R according to said method.

The processing device 2 comprises a controller 20 for executing the method. The controller 20 is a microprocessor an ASIC (application specific integrated circuit) or a FPGA (field programmable gate array). The processing device 2 is depicted as a single work station. Alternatively, in a not shown embodiment of the invention, the processing device 2 is a server or comprises one or more clustered computing devices.

Further the processing device 2 comprises a computer readable storage device 21 such as a hard disk, on which a computer program product 22 is loaded, wherein the computer program product 22 comprises program code means for executing the method described above, when executed by the controller 20 of the processing device 2.

The processing device 2 further comprises an interface 18 to a database system 23, which according to this embodiment of the invention is a server. The database system comprises a computer readable storage device 24 such as a hard disk, on which the database 8 is stored. This database system is suited to be connected (interfaced) to a number of not shown processing devices 2, for example by means of the internet, such that user of those processing devices 2 share the database 8.

In a not shown alternative, the processing device 2 is included in the database system 23. The database system 23 comprises a computer readable storage device 24.

By means of the displaying device 4 further instructions are provided for a user of the MRI-Scanner 10. Exemplarily, such an instruction is a rate of a contrast medium injection into the patient and/or the preferred sequence when the contrast medium should be injected.

The invention is not limited to the embodiment described above. Rather, other variants of the invention can also be derived therefrom without departing from the scope of the invention. In particular all the individual features described in connection with the exemplary embodiments can also be combined with one another in another manner without departing from the subject matter of the invention.

## Claims

1. Method for acquiring magnetic resonance image data (D) for image-guided radiotherapy,
- wherein a number of imaging goals (U) are provided,
- wherein for each imaging goal (U) a magnetic resonance imaging protocol (R)is selected,
- wherein for each selected magnetic resonance imaging protocol (R) a number of parameters (A) for said protocol (R) is adjusted according to the respective imaging goal (U), and
- wherein the magnetic resonance image data (D) are acquired with said magnetic resonance imaging protocols (R) and with said parameters (A).

2. The method according to claim 1,
wherein a database (8) is provided, which comprises information, assigning a magnetic resonance imaging protocol (R) and a number of the parameters (A) for said protocol (R) to each of a number of imaging goals (U).

3. The method according to claim 2,
wherein the magnetic resonance imaging protocol (R) is selected dependent on the respective imaging goal (U) by means of the database (8).

4. The method according to one of the claims 1 to 3, wherein each imaging goal (U) is converted into a number of first metrics (F) and a range for each of the first metrics (F).

5. The method according to claim 4,
wherein boundary conditions (B) are provided, and wherein the range of each of the first metrics (F) is adjusted according to said boundary conditions (B).

6. The method according to claim 4 or 5,
wherein within adjusting the parameters (A) for a magnetic resonance imaging protocol (R) according to the imaging goals (U),
- the parameters(A), which are assigned to the respective protocol (R) in the database, are obtained from the database (8),
- a number of second metrics (S) as a function of said parameters (A) is determined, the second metric (S) representing an expected first metric (F) for using said parameters (A),
- the range of each of the first metrics (F) are compared to the respective second metric (S), and
- said parameters (A) are altered, if the one of the second metrics (S) doesn't comply with the range of the respective first metric (F).

7. The method according to one of the claims 2 to 6, wherein an information is stored in the database (8), which assigns protocols (R) and parameters (A) used for acquiring the magnetic resonance image data (D) to the respective imaging goal (U).

8. Processing Device (2), comprising a controller (20) for executing the method according to one of the claims 1 to 7.

9. Database system (23), comprising
- a computer readable storage device (24), on which a database (8) is stored, wherein the database (8) comprises information, which assign a magnetic resonance imaging protocol (R) and parameters (A) for said protocol (R) to each of a number of imaging goals (U), and
- an interface (18) for a processing device (2) according to claim 8.

10. Computer program product (22), which is loadable into a computer readable storage device (21) of the processing device (2), with program code means for executing the method according to one of the claims 1 to 7.

## Amended claims

### Amended claims in accordance with Rule 137(2) EPC.

1. Method for acquiring magnetic resonance image data (D) for image-guided radiotherapy,
- wherein a number of imaging goals (U) are provided,
- wherein for each imaging goal (U) a magnetic resonance imaging protocol (R)is selected,
- wherein for each selected magnetic resonance imaging protocol (R) a number of parameters (A) for said protocol (R) is adjusted according to the respective imaging goal (U),
- wherein the magnetic resonance image data (D) are acquired with said magnetic resonance imaging protocols (R) and with said parameters (A), wherein
- a database (8) is provided, which comprises information, assigning a magnetic resonance imaging protocol (R) and a number of the parameters (A) for said protocol (R) to each of a number of imaging goals (U), wherein
- the magnetic resonance imaging protocol (R) is selected dependent on the respective imaging goal (U) by means of the database (8),
**characterized in that**
within adjusting the parameters (A)
- each imaging goal (U) is converted into a number of first metrics (F) and a range for each of the first metrics (F),
- the parameters(A), which are assigned to the respective protocol (R) in the database, are obtained from the database (8),
- a number of second metrics (S) as a function of said parameters (A) is determined, the second metric (S) representing an expected first metric (F) for using said parameters (A),
- the range of each of the first metrics (F) are compared to the respective second metric (S), and
- said parameters (A) are altered, if the one of the second metrics (S) doesn't comply with the range of the respective first metric (F).

2. The method according to claim 1,
wherein boundary conditions (B) are provided, and wherein the range of each of the first metrics (F) is adjusted according to said boundary conditions (B).

3. The method according to claim 1 or 2,
wherein the parameters (A) are altered according to an interactive, non linear optimization-algorithm.

4. The method according to one of the claims 1 to 3, wherein an information is stored in the database (8), which assigns protocols (R) and parameters (A) used for acquiring the magnetic resonance image data (D) to the respective imaging goal (U).

5. Processing Device (2), comprising a controller (20) for executing the method according to one of the claims 1 to 4.

6. Database system (23), comprising
- a computer readable storage device (24), on which a database (8) is stored, wherein the database (8) comprises information, which assign a magnetic resonance imaging protocol (R) and parameters (A) for said protocol (R) to each of a number of imaging goals (U), and
- the processing device (2) according to claim 8.

7. Computer program product (22), which is loadable into a computer readable storage device (21) of the processing device (2), with program code means for executing the method according to one of the claims 1 to 4.
